# EUROPÄISCHE PATENTSCHRIFT

(11) **EP 0 513 004 B1**
(45) Veröffentlichungstag und Bekanntmachung des Hinweises auf die Patenterteilung: **03.08.1994**
(21) Anmeldenummer: 91900246.9
(22) Anmeldetag: 28.11.1990
(51) Int. Cl.: G01N 25/08, G01N 27/18, G01N 33/28, B60T 17/22

(54) **VORRICHTUNG ZUR ERMITTLUNG DER BESCHAFFENHEIT EINER DRUCKÜBERTRAGUNGSFLÜSSIGKEIT**
DEVICE FOR DETERMINING THE NATURE OF A HYDRAULIC FLUID
DISPOSITIF DE DETERMINATION DE LA QUALITE D'UN LIQUIDE DE TRANSMISSION DE PRESSION

(30) Priorität: 31.01.1990 DE 4002792
(43) Veröffentlichungstag der Anmeldung: 19.11.1992
(73) Patentinhaber: ALFRED TEVES GmbH, 60441 Frankfurt (DE); PHYWE SYSTEME GMBH, 37079 Göttingen (DE)
(72) Erfinder: KLEIN, Hans-Christof, D-6234 Hattersheim (DE); LOHBERG, Peter, D-6382 Friedrichsdorf (DE); KRAUSE, Hans, Joachim, D-3400 Göttigen/Elliehausen (DE); MAY, Arno, D-3400 Göttingen (DE); OBERDORFER, Dietmar, D-3400 Göttingen (DE); PLÜQUETT, Ulrich, D-3400 Göttingen (DE)
(86) Internationale Anmeldenummer: EP9002037
(87) Internationale Veröffentlichungsnummer: WO9111707

(56) Entgegenhaltungen:
- EP-A- 0 108 962
- EP-A- 0 208 096
- EP-A- 0 280 229

## Beschreibung

Die Erfindung bezieht sich auf eine Vorrichtung zur Ermittlung der Beschaffenheit einer Druckübertragungsflüssigkeit, insbesondere zur Kontrolle oder Überwachung des Gebrauchswertes oder der Siedetemperatur einer hygroskopischen Bremsflüssigkeit, mit einem elektrisch beheizbaren Sensorelement, das in die zu untersuchende Flüssigkeit eintaucht, mit einer Stromquelle, die einen elektrischen Strom konstanter Amplitude liefert und mit einer Meßeinrichtung, mit der der Spannungsabfall über dem Sensorelement beim Einspeisen des elektrischen Stromes abgreifbar und der temperaturabhängige Widerstand des Sensorelementes ermittelbar ist, wobei das Sensorelement derart gestaltet und dimensioniert und der Heizstrom derart bemessen ist, daß sich nach dem Aufheizen des Sensorelementes für die Dauer der Messung eine stabile Zellularkonvektion in einem unterhalb der Siedetemperatur der Flüssigkeit liegenden Temperaturbereich einstellt, und wobei der Spannungsabfall über dem Sensorelement als Maß für die Beschaffenheit der Flüssigkeit auswertbar ist.

Eine derartige Vorrichtung ist aus der Offenlegungsschrift DE 35 22 774 A1 bekannt. Das Sensorelement dieser Vorrichtung ist derart gestaltet und die Stromversorgung derart ausgelegt, daß sich zum Zeitpunkt der Messung eine stabile Zellularkonvektion einstellt. Dies setzt ein Aufheizen auf eine bestimmte Temperatur voraus, die in einem unterhalb der Siedetemperatur der zu untersuchenden Flüssigkeit liegenden Temperaturbereich liegt. Ein Aufheizen des Sensorelementes auf die Siedetemperatur der zu untersuchenden Flüssigkeit ist dabei unbedingt zu vermeiden.

Nach dieser Offenlegungsschrift wird vorgeschlagen, das Sensorelement in Form eines Hohlkörpers mit offener, durchbrochener Wandung, in Form einer Hohlwendel, einer perforierten Röhre und der dergl. auszubilden, um das Ausbilden von Konvektionszellen bzw. der stabilen Zellularkonvektion während der Meßdauer zu begünstigen. Solche Sensorelemente sind jedoch relativ aufwendig. Dies kommt besonders dann zum Tragen, wenn ein stationärer Einbau in ein Fahrzeug bzw. in jede Radbremse eines Fahrzeugs vorgesehen ist. Auch sind in einem solchen Fall die Anforderungen an die mechanische Stabilität des Sensorelementes hoch.

Der Erfindung liegt daher die Aufgabe zugrunde, ein mechanisch stabiles und dennoch mit geringem Aufwand herzustellendes Sensorelement für eine Vorrichtung der eingangs genannten Art zu entwickeln, das die Ausbildung einer stabilen Zellularkonvektion während des Meßvorganges begünstigt.

Es hat sich gezeigt, daß diese Aufgabe überraschenderweise mit einem Sensorelement zu lösen ist, das in der sehr einfachen Form eines beidseitig eingespannten Linearleiters, z.B. eines kurzen Drahtstückes, ausgebildet ist. Das Sensorelement kann auch aus mehreren Linearleitern dieser Art, die parallel oder in Serie geschaltet sind, bestehen.

Die Anordnung muß derart getroffen werden, daß sich an allen Linearleitern Konvektionszellen ausbilden können.

Nach einem vorteilhaften Ausführungsbeispiel der Erfindung besteht das Sensorelement aus einer Platin/Iridiumlegierung der Zusammensetzung 90%/10%. Das Sensorelement kann eine Länge von 10 - 30 mm, vorzugsweise 15 - 20 mm, aufweisen und einen Durchmesser von 30 - 80 µm, vorzugsweise 40 - 60 µm besitzen.

Nach einem weiteren vorteilhaften Ausführungsbeispiel der Erfindung ist das Sensorelement in der zu untersuchenden Flüssigkeit annähernd senkrecht angeordnet. Es kann jedoch auch mit vorgegebener Neigung oder mit einer zur Einstellung der Meßcharakteristik veränderlichen Neigung in die Flüssigkeit eingesetzt sein. Schließlich ist es in manchen Fällen noch günstig, wenn das Sensorelement, zumindest teilweise von einem koaxialen Rohr umgeben ist, das beidseitig offen ist und die Ausbildung eines Hüllstromes bzw. einer stabilen Zellularkonvektion zwischen dem Linearleiter und dem Rohr zuläßt. Es derartiges Sensorelement ist gegen Strömungsbewegungen in der zu untersuchenden Flüssigkeit relativ unempfindlich. Zur Konstruktion handgeführter Meßsonden ist eine solche Ausbildung ebenfalls von Vorteil.

Weitere Merkmale, Vorteile und Anwendungsmöglichkeiten der Erfindung gehen aus der folgenden Beschreibung von Ausführungsbeispielen anhand der beigefügten Abbildungen hervor.

Es zeigen
- Fig. 1: im Diagramm den prinzipiellen Verlauf der Temperatur bzw. des ohmschen Widerstandes über den Strom bei einem Sensorelement der erfindungsgemäßen Art,
- Fig. 2: den grundsätzlichen Aufbau einer Meßschaltung für die erfindungsgemäße Vorrichtung,
- Fig. 3: in schematisch vereinfachter Darstellung ein Sensorelement für die erfindungsgemäße Vorrichtung,
- Fig. 4 a - e: in Prinzipdarstellung verschiedene Ausführungsformen des Sensorelementes.

Die erfindungsgemäße Vorrichtung beruht auf der Siedetemperatur-Bestimmung mit Hilfe eines Sensorelementes, das derart ausgebildet ist und derart aufgeheizt wird, daß sich eine stabile Zellularkonvektion - jedoch kein Sieden - einstellt. Eine solche Zellularkonvektion tritt ein, wenn das als Konvektionskörper verwendete Sensorelement in dem unmittelbar angrenzenden Flüssigkeitsraum eine Wärmemenge erzeugt, die nicht mehr durch "laminare Konvektion" (abströmende Wärmeleitung) schnell genug an das umgebende Gesamtvolumen an Flüssigkeit weitergeleitet werden kann, die jedoch noch keine Siedeblasen erzeugt. Es bilden sich dann Grenzschichten aus, die das Heizelement in geringem Abstand wie ein Hüllstrom umgeben. Innerhalb einer "Zelle" entsteht ein Wärmerückstau bis zum Heizelement; die Zelle selbst kann nach außen in den Flüssigkeitsraum durch laminare Konvektion gerade so viel Wärme abgeben, wie in diesem Raum pro Zeiteinheit aufgenommen und verteilt werden kann. Das Heizelement und sein Konvektionszellenumfeld verhalten sich damit wie ein gemeinsames Heizgebilde, das sich in bezug auf laminare Konvektionsverhältnisse mit der Restflüssigkeit im Zustand der thermischen Leistungsanpassung befindet. Die Grenzschicht bleibt stabil, solange die Rückstautemperatur an der Innenseite der Grenzschicht um einen gewissen Betrag höher ist als an der Außenseite in der Restflüssigkeit. Wird die Flüssigkeit über die Temperatur der Konvektionszellenbildung hinaus erhitzt, bilden sich keine Konvektionszellen mehr aus und eine Siedetemperaturbestimmung mit Hilfe einer Vorrichtung der erfindungsgemäßen Art ist nicht mehr möglich. Die innerhalb der Konvektionszellen auftretende Rückstau-Temperaturverteilung ist u.a. abhängig von der Flüssigkeitsbewegung in der Zelle. Diese Bewegung ist wiederum abhängig von der Dichte und Zähigkeit (Viskosität) der Flüssigkeit sowie von Auftriebserscheinungen.

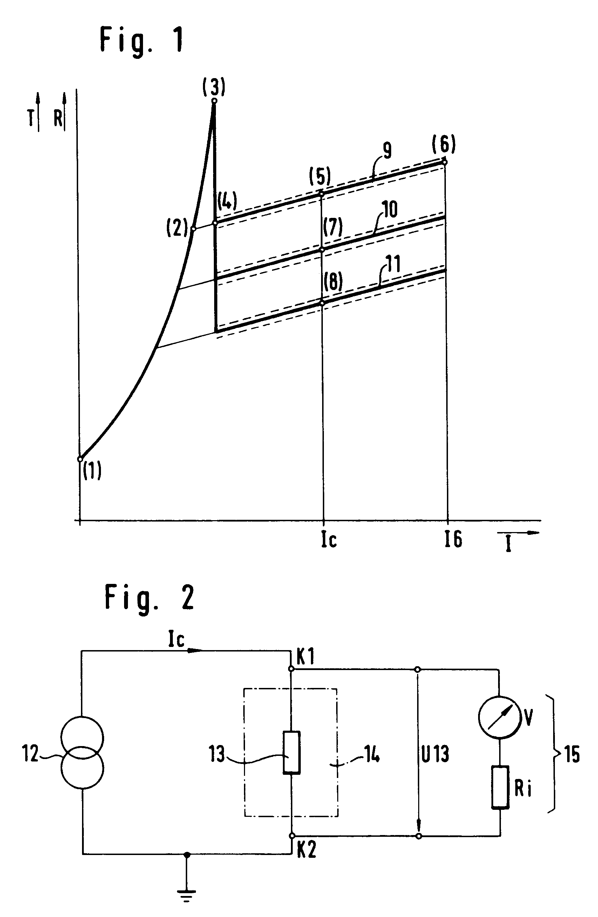

Zur Bestimmung der Siedetemperatur mit Hilfe der erfindungsgemäßen Vorrichtung wird der veränderliche Heizwiderstand des Sensorelementes infolge der Rückstautemperatur an der Grenzschicht zwischen der Heizeroberfläche und der Zellenflüssigkeit ausgewertet. Bei hygroskopischen Bremsflüssigkeiten bewirkt nun der Versatz mit Wasser eine spezifische Veränderung von Dichte unbd Viskosität sowie damit der Rückstautemperatur. Diese Änderung wird zur Bestimmung der Siedetemperatur ausgewertet. Das Diagramm nach Fig. 1 dient zur Veranschaulichung der Vorgänge. Dargestellt ist der Verlauf der Rückstautemperatur T oder des entsprechenden elektrischen Widerstandes R des Sensorelementes, mit dem diese Temperatur bestimmt wird, in Abhängigkeit von dem ansteigenden Heizstrom I. Ausgehend von der Umgebungstemperatur (1) steigt die Temperatur zunächst über einen Wert (2) hinaus parabelförmig bis zu einem Maximum (3) an. Dieser Verlauf entspricht dem Bereich mit ausschließlich laminarer Konvektion. Die Kennlinie nimmt dann einen ausgeprägten negativen Verlauf (3)-(4) und verläuft dann weiter mit geringer, annähernd konstanter Steigung bis zum Punkt (6). Der Kennlinienabschnitt (4)-(6) ist charakteristisch für den Bereich zellularer Konvektion, der erfindungsgemäß zur Bestimmung der Siedetemperatur der zu untersuchenden Flüssigkeit ausgewertet wird. (5) markiert einen günstigen Betriebsarbeitspunkt.

Das Dreieck (2)-(3)-(4) wird als Bifurkationszone bezeichnet, weil die Kennlinie mit ansteigender Heizenergie stets den Weg (2)-(3)-(4) nimmt, umgekehrt jedoch den Verlauf (4)-(2) nimmt, unter Auslassung des Punktes (3). Die Bifurkationszone charakterisiert den Bereich der beginnenden Ausbildung der zuvor beschriebenen Grenzschicht. Sensorelemente bzw. Heizelemente, die einen derartigen Kennlinienverlauf zeigen, sind grundsätzlich als Konvektionskörper bzw. Sensorelemente für die erfindungsgemäße Vorrichtung geeignet. Eine ausgeprägte Bifurkationszone ist in der Regel ein Indiz für eine vorteilhafte Gestaltung des Sensorelementes.

In Abhängigkeit von dem Wasseranteil in der zu untersuchenden Flüssigkeit ändert sich das Kennlinien-Plateau (4)-(6). Die Kennlinien 10 und 11 gelten für Flüssigkeiten, deren Siedepunkt im Vergleich zur Kennlinie 9 - infolge höheren Wassergehaltes - geringer ist. Beim Einspeisen eines bestimmten, konstanten Stromes Ic, wie dies bei der erfindungsgemäßen Vorrichtung geschieht, stellt sich anstelle des Arbeitspunktes 5 im Falle der Kennlinie 9 nun ein Arbeitspunkt (7) oder (8) (Kennlinie 10 bzw. 11) ein, zu dem ein bestimmter elektrischer Widerstand bzw. ein bestimmter Spannungsabfall über dem Sensorelement gehört.

Fig. 2 zeigt den grundsätzlichen Aufbau einer für die erfindungsgemäße Vorrichtung geeigneten Meßanordnung. Danach besteht die Meßanordnung im wesentlichen aus einer Stromquelle 12, die einen Gleich- oder Wechselstrom Ic konstanter Amplitude liefert, aus dem Sensorelement 13, das in die zu untersuchende Flüssigkeit, deren Volumen mit 14 symbolisiert ist, eingetaucht ist, sowie schließlich aus einem hochohmigen Spannungsmesser 15, der an den Klemmen K1,K2 angeschlossen ist.

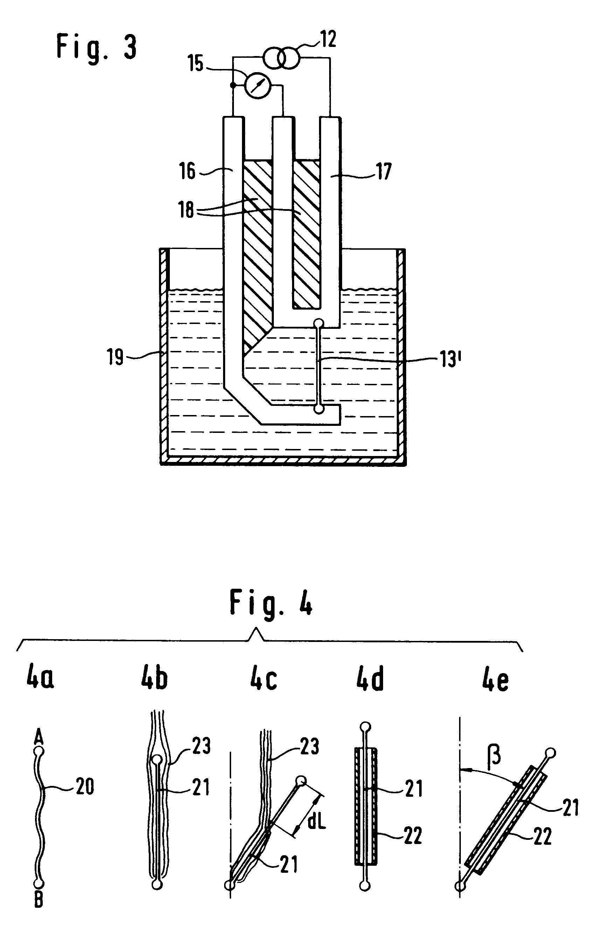

Fig. 3 veranschaulicht ein konkretes Beispiel einer Vorrichtung der erfindungsgemäßen Art mit einer Meßschaltung nach Fig. 2. Der als Sensorelement dienende Linearleiter 13' besteht in diesem Ausführungsbeispiel aus einem kurzen, beidseitig eingespannten Drahtstück, zu dessen Herstellung eine Platin-Iridium-Legierung Pt/Ir (90%/10%) verwendet wurde. Das Sensorelement 13' besitzt hier eine Länge von 10 - 15 mm und einen Druchmesser von 50 µm. Die Lange ist so gewählt, daß sich ein ohmscher Widerstand im Bereich von 1,7 - 2,3 Ohm ergibt. Der Betriebsstrom zur Erzeugung einer stabilen Zellularkonvektion liegt dann im Bereich von 1 Ampere.

In dem Ausführungsbeispiel nach Fig. 3 ist der Linearleiter 13' der erfindungsgemäßen Vorrichtung zwischen zwei Elektroden 16,17 aus Neusilberblech eingespannt, die in einem elektrisch nichtleitenden flachen Stützkörper 18 eingebettet sind. In Anlehnung an Fig. 2 wurde die Konstantstromquelle mit 12, der Spannungsmesser mit 15 symbolisiert. Das Sensorelement 13' ist mit den Elektroden 16,17 durch eine Punktschweißung verbunden. Das Sensorelement ist in einen mit der zu untersuchenden Flüssigkeit gefüllten Behälter 10 eingetaucht.

Es ist durchaus auch möglich, zwei oder mehrere gleichartige, einfach strukturierte Sensorelemente zusammenzufügen und elektrisch parallel oder in Serie geschaltet zu betreiben. Die räumliche Anordnung und der Abstand der einzelnen Sensorelemente voneinander müßten derart gewählt werden, daß sich die erfindungswesentliche Zellularkonvektion ausbilden kann. Beispielsweise könnten weitere gleichartige Linearleiter 13' zwischen den Elektroden 16,17 parallel angeordnet und an die Elektroden angeschweißt werden.

Die grundsätzliche Gestaltung und Anordnung besonders einfacher Linearleiter sind in Fig. 4 zusammengestellt. Nach Fig. 4a besteht das Sensorelement aus einem kurzen Linearleiter 20, der theoretisch als eine entartete Wendel aufgefaßt werden kann, die derart gestreckt ist, daß kein umschlossener Innenraum mehr verbleibt, wobei im Extremfall ein gewellter oder gar ein gerader Linienleiter entsteht. Bei vorgegebenem ohmschen Widerstand eines wendelartigen Heiz- oder Sensorelementes steht der Betriebsstrombedarf zur Erzeugung einer Zellularkonvektion in einem bestimmten Verhältnis zur Steigung der Wendel. Bei einer engen Wendel wird ein Minimum an Betriebsstrom benötigt, bei einer zum gestreckten Leiter entarteten Wendel ein etwas höherer Wert. Eine gestreckte Wendel mit geringem Verhältnis von Wendelinnendurchmesser zu Drahtdurchmesser verfügt jedoch über eine bessere mechanische Eigensteifigkeit, was für die praktische Anwendung eines Sensorelementes nach der Erfindung von großem Vorteil ist. Fig. 4a zeigt eine derartig gestreckte, entartete Wendel 20. Die beiden Endpunkte A und B, die durch Materialverstärkung und dadurch erhöhten Leitwert gegeben sind, begrenzen die wirksame Länge des Sensorelementes. Die Fig. 4 b und c zeigen Linearleiter 21 in Form von vollständig gestreckten Linienleitern. In Fig. 4b ist das Sensorelement senkrecht angeordnet, während in Fig. 4c die Achse um einen bestimmten Winkel gegenüber der Senkrechten gekippt ist. In beiden Fällen ist der Schlierenverlauf des Hüllstromes 23 angedeutet. In Abhängigkeit von der Neigung des Linienleiters 21, der das Sensorelement darstellt, und der damit verbundenen Wirkung der Auftriebskräfte auf den Hüllstrom 23 verbleibt ein bestimmter Teil des Linearleiters 21 im Wirkungsbereich des Hüllstromes 23. Durch Änderungen bzw. Einstellung der Neigung ist folglich die Charakteristik des als Meßkörper dienenden Sensorelementes veränderbar und läßt sich an bestimmte Anforderungen anpassen. So ist in Fig. 4b das Gesamtsensorelement umhüllt; in Fig. 4c verbleibt eine Länge dL ohne Hüllstrom. Dieser Umstand kann dazu ausgenutzt werden, dem Kurvenverlauf des Linienabschnittes (4)-(6) in Fig. 1 eine veränderte Charakteristik zu geben.

In den Ausführungsvarianten nach Fig. 4d und e ist der gestreckte Linienleiter 21, der das Sensorelement darstellt, mit einer Umhüllung bzw. einem koaxialen Rohr 22 umgeben, das beidseitig offen ist, so daß der Hüllstrom zwischen dem Linienleiter 21 und dem Rohr 22 geführt wird. Derartige Sensorelemente sind gegen zusätzliche Strömungsbewegungen in der Prüfungsflüssigkeit weniger empfindlich als Sensorelemente 21 ohne derartige Umhüllungen. Die Sensorelemente nach Fig. 4d und e sind zur Konstruktion handgeführter Sonden besonders geeignet. Für ortsfeste Systeme, z.B. zum Einbau in Kraftfahrzeugbremsen, genügen in der Regel die einfacheren Ausführungsformen nach den Fig. 4a - c.

## Patentansprüche

1. Vorrichtung zur Ermittlung der Beschaffenheit einer Druckübertragungsflüssigkeit, insbesondere der Siedetemperatur einer hygroskopischen Bremsflüssigkeit,
- mit einem elektrisch beheizbaren Sensorelement (13), das in die zu untersuchende Flüssigkeit eintaucht,
- mit einer Stromquelle (12), die einen elektrischen Strom (Ic) konstanter Amplitude liefert,
- mit einer Meßeinrichtung (15) zur Ermittlung des Spannungsabfalls über dem Sensorelement (13) beim Einspeisen des elektrischen Stromes und zur Ermittlung des temperaturabhängigen Widerstandes (R) des Sensorelementes (13),
- wobei das Sensorelement (13) derart gestaltet und der Heizstrom (I) derart bemessen ist, daß sich nach dem Aufheizen des Sensorelementes (13) für die Dauer der Messung eine stabile Zellularkonvektion in einem unterhalb der Siedetemperatur der Flüssigkeit liegenden Temperaturbereich einstellt, und
- wobei der Spannungsabfall (U13) über dem Sensorelement (13) als Maß für die Beschaffenheit der Flüssigkeit auswertbar ist,
dadurch **gekennzeichnet**,
- daß das Sensorelement (13) in Form mindestens eines beidseitig eingespannten Linearleiters (13',20,21), z.B. eines Drahtes, ausgebildet ist.

2. Vorrichtung nach Anspruch 1, dadurch **gekennzeichnet**,
daß das Sensorelement (13) aus mehreren, parallel oder in Serie geschalteten Linearleitern (13',20,21) besteht.

3. Vorrichtung nach Anspruch 1 oder 2, dadurch **gekennzeichnet**, daß der Linearleiter (13',20,21) aus einer Metallegierung, z.B. aus Pt/Ir (90%/10%), besteht.

4. Vorrichtung nach einem oder mehreren der Ansprüche 1 - 3, dadurch **gekennzeichnet**, daß der Linearleiter (13',20,21) eine Länge von 10 - 30 mm, insbesondere 15 - 20 mm, aufweist und einen Durchmesser von 30 - 80 µm, insbesondere 40 - 60 µm, besitzt.

5. Vorrichtung nach einem oder mehreren der Ansprüche 1 - 4, dadurch **gekennzeichnet**, daß das Sensorelement (13') bzw. der Linearleiter (13',20,21) in der zu untersuchenden Flüssigkeit annähernd senkrecht angeordnet ist.

6. Vorrichtung nach einem oder mehreren der Ansprüche 1 - 4, dadurch **gekennzeichnet**, daß der Linearleiter (21) mit einer vorgegebenen Neigung (β) in der zu untersuchenden Flüssigkeit angeordnet ist.

7. Vorrichtung nach Anspruch 6, dadurch **gekennzeichnet**, daß die Neigung, mit der der Linearleiter (21) in der Flüssigkeit angeordnet ist, zur Einstellung der Meßcharakteristik des Sensorelementes (13) veränderbar ist.

8. Vorrichtung nach einem oder mehreren der Ansprüche 1 - 7, dadurch **gekennzeichnet**, daß der Linearleiter (21) zumindest teilweise von einem koaxialen Rohr (22) umgeben ist, das beidseitig offen ist und die Ausbildung eines Hüllstromes (23) bzw. einer stabilen Zellularkonvektion zwischen dem Linearleiter (21) und dem Rohr (22) zuläßt.

## Claims

1. An apparatus for determining the condition of a pressure transmitting fluid, especially for determining the boiling temperature of a hygroscopic brake fluid,
- including an electrically heatable sensor element (13) immersed into the fluid to be tested,
- including a power source (12) supplying an electric current (Ic) of constant amplitude,
- including a measuring device (15) for detecting the voltage drop on the sensor element (13) upon delivery of the electric current and for determining the temperature-responsive resistance (R) of the sensor element (13),
- with the sensor element (13) being so shaped and the heating current (I) being so dimensioned that after heating the sensor element (13), a stable cellular convection arises for the duration of the measurement in a temperature range below the boiling temperature of the fluid, and
- with the voltage drop (U13) on the sensor element (13) being analyzable as a criterion for the condition of the fluid,
**characterized** in that
- the sensor element (13) is in the form of at least one linear conductor (13', 20, 21), for example a wire, clamped in on either side.

2. An apparatus as claimed in claim 1,
**characterized** in that the sensor element (13) is formed of a plurality of linear conductors (13', 20, 21) which are connected in parallel or in series.

3. An apparatus as claimed in claim 1 or claim 2,
**characterized** in that the linear conductor (13', 20, 21) is made of a metal alloy, for instance Pt/Ir (90%/10%).

4. An apparatus as claimed in any one or more of claims 1 to 3,
**characterized** in that the linear conductor (13', 20, 21) has a length of between 10 and 30 mm, in particular between 15 and 20 mm, and has a diameter of between 30 and 80 µm, in particular between 40 and 60 µm.

5. An apparatus as claimed in any one or more of claims 1 to 4,
**characterized** in that the sensor element (13') and the linear conductor (13', 20, 21), respectively, is disposed in the fluid to be tested in a direction approximately vertical.

6. An apparatus as claimed in any one or more of claims 1 to 4,
**characterized** in that the linear conductor (21) is disposed in the fluid to be tested at a predetermined inclination (β).

7. An apparatus as claimed in claim 6,
**characterized** in that the inclination at which the linear conductor (21) is disposed in the fluid is variable for adjusting the measuring characteristic of the sensor element (13).

8. An apparatus as claimed in any one or more of claims 1 to 7,
**characterized** in that the linear conductor (21), at least in part, is surrounded by a coaxial tube (22) which is open on both sides and permits the formation of an enveloping stream (23) and a stable cellular convection, respectively, between the linear conductor (21) and the tube (22).

## Revendications

1. Dispositif permettant de déterminer l'état d'un liquide de transmission de pression, notamment la température d'ébullition d'un liquide de frein hygroscopique,
- comprenant un élément capteur (13) a chauffage électrique, plongé dans le liquide à examiner,
- une source de courant (12) fournissant un courant électrique (Ic) à amplitude constante,
- un dispositif de mesure (15) permettant de prélever la chute de tension a travers l'élément capteur (13) lorsque l'appareil est mis sous tension, et de déterminer la résistance (R) dépendant de la température de l'élément capteur (13),
- l'élément capteur (13) étant configuré et dimensionné, et le courant de chauffage (I) étant réglé, de telle sorte qu'après l'échauffement de l'élément capteur (13), on obtienne, pour la durée de la mesure, une convection cellulaire stable s'effectuant dans une plage de température située en dessous du point d'ébullition du liquide, et
- la chute de tension (U13) créée à travers l'élément capteur (13) pouvant être exploitée comme une mesure de l'état du liquide,
caractérisé en ce que
- l'élément capteur (13) est réalisé sous forme d'au moins un conducteur linéaire (13' , 20, 21) , par exemple d'un fil métallique serré aux deux extrémités.

2. Dispositif selon la revendication 1, caractérisé en ce que l'élément capteur (13) se compose de plusieurs conducteurs linéaires (13' , 20, 21) branchés en parallèle ou en série.

3. Dispositif selon l'une des revendications 1 ou 2, caractérisé en ce que le conducteur linéaire (13', 20, 21) est constitué d'un alliaqe métallique, par exemple de Pt/Ir (90%/10%).

4. Dispositif selon l'une ou plusieurs des revendications 1 à 3, caractérisé en ce que le conducteur linéaire (13', 20, 21) présente une longueur de 10 à 30 mm, notamment une longueur de 15 à 20 mm, et un diamètre de 30 à 80 µm, notamment un diamètre de 40 à 60 µm.

5. Dispositif selon l'une ou plusieurs des revendications 1 à 4, caractérisé en ce que l'élément capteur (13') **ou** le conducteur linéaire (13', 20, 21) est disposé à peu prés verticalement dans le liquide à examiner.

6. Dispositif selon l'une ou plusieurs des revendications 1 à 4, caractérisé en ce que le conducteur linéaire (21) est disposé de façon inclinée selon un angle prédéfini (β) dans le liquide à examiner.

7. Dispositif selon la revendication 6, caractérisé en ce que l' inclinaison selon laquelle le conducteur linéaire (21) est disposé dans le liquide peut étre modifiée de manière à permettre le réglage de la caractéristique de mesure de l'élément capteur (13).

8. Dispositif selon l'une ou plusieurs des revendications 1 à 7, caractérise en ce que le conducteur linéaire (21) est au moins partiel lement entouré par un tube coaxial (22) ouvert des deux côtés et permettant d'établissement d'un courant enveloppant (23) **ou** d'une convection cellulaire stable entre le conducteur linéaire (21) et le tube (22).
